Europäisches Patentamt

European Patent Office

Office européen des brevets

(11) Publication number: **0 242 904**
**A2**

# (12) EUROPEAN PATENT APPLICATION

(21) Application number: 87200608.5

(22) Date of filing: 30.03.87

(51) Int. Cl.⁴: **C01B 33/28** , **C10G 45/64** , **C10G 25/03** , **C07C 2/00**

(30) Priority: 23.04.86 GB 8609879

(43) Date of publication of application:
**28.10.87 Bulletin 87/44**

(84) Designated Contracting States:
**BE CH DE ES FR GB IT LI NL SE**

(71) Applicant: **SHELL INTERNATIONALE RESEARCH MAATSCHAPPIJ B.V.**
**Carel van Bylandtlaan 30**
**NL-2596 HR Den Haag(NL)**

(72) Inventor: **Van Erp, Willibrord Adelbert**
**Badhuisweg 3**
**NL-1031 CM Amsterdam(NL)**
Inventor: **Seelen-Kruijssen, Josepha Maria Elisabeth**
**Badhuisweg 3**
**NL-1031 CM Amsterdam(NL)**
Inventor: **Hoek, Arend**
**Badhuisweg 3**
**NL-1031 CM Amsterdam(NL)**
Inventor: **Huizinga, Tom**
**Badhuisweg 3**
**NL-1031 CM Amsterdam(NL)**

(74) Representative: **Aalbers, Onno et al**
**P.O. Box 302**
**NL-2501 CH The Hague(NL)**

(54) A crystalline aluminium silicate, its preparation and its use as catalyst (carrier).

(57) A crystalline aluminium silicate obtainable by maintaining an aqueous alkaline starting mixture comprising one or more silicon compounds, one or more aluminium compounds, one or more compounds of metals from Group 1a of the Periodic Table of the Elements (MX) and organic nitrogen compounds at an elevated temperature until crystalline aluminium silicate has formed and subsequently separating crystalline silicate from the mother liquor, in which starting mixture the various compounds are present in defined molar ratios. The crystalline aluminium silicate can be used as catalyst (carrier) for dewaxing hydrocarbon oils and preparing liquid hydrocarbons, and for separating hydrocarbons of different shape.

# A CRYSTALLINE ALUMINIUM SILICATE, ITS PREPARATION AND ITS USE AS CATALYST (CARRIER)

The invention relates to a crystalline aluminium silicate, its preparation and its use as catalyst (carrier). More particularly it relates to a crystalline aluminium silicate obtainable by a specific process.

Crystalline aluminium silicates, which can be distinguished from one another by certain characteristic lines occurring in their X-ray powder diffraction patterns can be prepared hydrothermally by maintaining an aqueous alkaline starting mixture comprising one or more silicon compounds, one or more aluminium compounds, one or more alkali metal compounds and one or more organic nitrogen compounds at an elevated temperature until the appropriate crystalline aluminium silicate has (have) formed and subsequently separating crystalline silicate from the mother liquor, usually followed by washing and drying.

The right selection of reaction components comprised in the aqueous mixture and their molar ratios is of major importance in preparing the desired crystalline aluminium silicates.

It is known that the microscopic texture (e.g. the crystal shape, size, degree of smoothness and the degree of agglomeration of the ultimate crystals) of a synthetic zeolite is influenced by the organic nitrogen compound(s) present in the starting mixture used to carry out the above-described hydrothermal process.

It is also known to prepare a composite crystalline aluminium silicate of which the X-ray powder diffraction pattern includes some of the characteristic lines of two different crystalline aluminium silicates by maintaining an aqueous starting mixture comprising one or more silicon compounds, one or more aluminium compounds, one or more alkali metal hydroxides, one or more alkali metal salts of mineral acids, a pyridine and an organic quaternary ammonium compound at an elevated temperature.

When producing larger amounts of one specific crystalline aluminium silicate on a (semi-)commercial scale it is highly desirable that the use of a relatively large amount of a rather difficult to prepare and thus expensive quaternary ammonium compound in the starting mixture can be reduced considerably.

It has now been found that from an aqueous alkaline starting mixture comprising as organic nitrogen compounds a pyridine and a relatively small amount of an organic quaternary ammonium compound a novel crystalline aluminium silicate of which the X-ray powder diffraction pattern includes characteristic lines of substantially one specific silicate can be reproduceably prepared, provided that the various compounds are present in the starting mixture in specific molar ratio ranges which are critical requirements.

The present invention therefore relates to a crystalline aluminium silicate which is obtainable by maintaining an aqueous alkaline starting mixture comprising one or more silicon compounds, one or more aluminium compounds, one or more compounds of metals from Group 1a of the Periodic Table of the Elements (MX) and organic nitrogen compounds at an elevated temperature until crystalline aluminium silicate has formed and subsequently separating crystalline silicate from the mother liquor, in which starting mixture the various compounds are present within the following molar ratios:

$RN : R_4NY = 1\text{-}100$,
$SiO_2 : R_4NY = 10\text{-}500$,
$SiO_2 : Al_2O_3 = 50\text{-}300$,
$SiO_2 : MX < 15$, and
$H_2O : SiO_2 = 5\text{-}100$,

RN representing a pyridine and $R_4NY$ representing an organic quaternary ammonium compound.

The invention furthermore relates to a process for the preparation of a crystalline aluminium silicate which comprises maintaining an aqueous alkaline starting mixture comprising one or more silicon compounds, one or more aluminium compounds, one or more compounds of metals from Group 1a of the Periodic Table of the Elements (MX) and organic nitrogen compounds at an elevated temperature until crystalline aluminium silicate has formed and subsequently separating crystalline silicate from the mother liquor, in which starting mixture the various compounds are present within the following molar ratios:

$RN : R_4NY = 1 - 100$,
$SiO_2 : R_4NY = 10 - 500$,
$SiO_2 : Al_2O_3 = 50 - 300$
$SiO_2 : MX < 15$, and
$H_2O : SiO_2 = 5 - 100$,

RN representing a pyridine and $R_4NY$ representing an organic quaternary ammonium compound.

The Periodic Table of the Elements referred to is stated in the "Handbook of Chemistry and Physics", 55th edition, CRC Press, Ohio, USA (1975).

Preferably RN represents a compound selected from the group consisting of pyridine, alkyl pyridines and (substituted alkyl) pyridines, wherein the alkyl-groups preferably comprise of from 1-4 carbon atoms, and amino pyridines; most preferably RN represents pyridine.

The groups R in $R_4NY$ preferably comprise four of the same or different alkyl-groups and/or substituted alkyl-groups, e.g. alkyl-groups comprising a hydroxy-and/or a halogen (e.g. bromine)-substituent; these alkyl-groups generally comprise of from 1-20, preferably of from 1-4 carbon atoms. The symbol Y in $R_4NY$ suitably represents an anion of a mineral acid or a hydroxyl ion. Preferably $R_4NY$ represents tetrapropyl ammonium hydroxide, a suitable alternative therefore being tetraethyl ammonium bromide.

The above-defined organic nitrogen compounds RN and $R_4NY$ are preferably present in the starting mixture in a molar ratio from 5-80, and most preferably from 10-75, which means that the molar quantity of compound $R_4NY$ is preferably substantially smaller than the employed molar quantity of compound RN. The molar ratio in which $SiO_2$ and $R_4NY$ are present in the starting mixture is preferably from 20-400, and most preferably from 30-300.

The compound MX in the starting mixture preferably represents at least one of $M_nZ$ and at least one of MOH in which M represents an alkali metal ion and $Z^{n-}$ represents an anion of a mineral acid (n satisfying the electroneutrality of the compound $M_nZ$); most preferably M represents a sodium ion. The compounds $SiO_2$ and MOH are suitably present in the starting mixture in a molar ratio of from 5.2-7.8, preferably of from 5.6-7.0. In any case the aqueous starting mixture has an alkaline character which means that the pH of the starting mixture is greater than 7.

The compounds $SiO_2$, $Al_2O_3$ and $H_2O$ are preferably present in the starting mixture in the following molar ratios: $SiO_2:Al_2O_3$ = 65-200, and $H_2O:SiO_2$ = 8-60.

In the starting mixture used in the preparation of a crystalline aluminium silicate according to the present invention various silicon-and aluminium compounds may be present. Suitable silicon compounds include water glass and amorphous silica, while suitable aluminium compounds include aluminium sulphate and sodium aluminate. Suitable alkali metal salts of mineral acids include sulphates, nitrates and phosphates. It is not necessary, however, that the above-mentioned compounds are added to the aqueous starting mixture in that form. They may also be formed from other reaction components, for instance from water glass and sulphuric acid. A very suitable starting mixture comprises amorphous silica, aluminium sulphate, sodium hydroxide, sodium sulphate, pyridine, water and either tetrapropyl ammonium hydroxide or tetraethyl ammonium bromide.

The crystalline aluminium silicates are suitably prepared in the manner as described hereinbefore by maintaining the starting mixture, usually under autogenous pressure, at an elevated temperature, preferably from 100-250 °C, for 24-190 hours, preferably from 30-120 hours, under stirred conditions, until the appropriate crystalline aluminium silicate has formed and subsequently separating crystalline silicate from the mother liquor (e.g. by means of filtration or centrifugation), washing the crystalline silicate thus obtained and drying (suitably at a temperature of from 100-200 °C), optionally followed by calcining at a temperature of from 200-600 °C.

The present invention relates in particular to a crystalline aluminium silicate having a characteristic X-ray diffraction pattern as shown in Table A and discussed hereinafter.

The crystalline aluminium silicates as synthesized contain alkali metal. An alkali metal content of more than 0.1% by weight is undesirable, however, when the crystalline aluminium silicates are to be used as catalyst or catalyst carrier in the catalytic dewaxing of hydrocarbon oils, to which process the present invention also relates. In order to reduce the alkali metal content of the silicates to less than 0.1% by weight and in particular to less than 0.01% by weight, the silicates are suitably contacted once or several times with an aqueous solution which comprises ammonium ions. From the $NH_4^+$ aluminium silicates obtained in this manner the $H^+$ aluminium silicates can be prepared by calcination. The present invention furthermore relates to a process for the catalytic conversion of a hydrocarbons-containing feed at elevated temperature and pressure, in which process a catalyst is employed which contains a crystalline aluminium silicate as defined hereinbefore.

If desired, the performance (e.g. catalytic activity and stability) in a catalytic dewaxing process of the aluminium silicates which have been treated as described hereinbefore can be further improved by using them simultaneously as carrier for one or more catalytically active metals from Groups 6b, 7b and 8 of the Periodic Table of the Elements or compounds thereof. Of particular interest are the metals molybdenum, tungsten, chromium, iron, nickel, cobalt, platinum, palladium, ruthenium, rhodium, osmium and irridium; platinum and/or palladium are preferably used because it appears that their presence on the silicates reduces the undesired formation of aromatic compounds, leading to such a high dewaxed product quality that further hydrotreating of the dewaxed product is no longer necessary. The metals or their compounds may be deposited on the silicates by means of any process for the preparation of catalysts known in the art, such as impregnation, ion-exchange or precipitation.

The metal-loaded aluminium silicates suitably comprise from 1-50% by weight, preferably from 2-20% by weight, of a non-noble metal of Group 6b, 7b and/or 8; noble metals of Group 8 are suitably present in the aluminium silicate catalysts in an amount from 0,001-5% by weight, preferably from 0.01-2% by weight.

When the crystalline aluminium silicates are used as catalysts or catalyst carriers they should generally be available in the form of particles with a diameter of 0.5-5 mm. The crystalline aluminium silicates according to the present invention are normally obtained in the form of a fine powder and may be shaped to form particles of a larger size, for instance by pressing or extruding. During shaping the aluminium silicates may be combined with a binder material, suitably in a weight ratio from 1-10; preferably a binder material is used which contains no or only very little alkali metal such as alumina. The binder material may also exert catalytic activity if so desired. Catalytically active metals, especially noble metals, are suitably deposited on the binder material (e.g. by means of ion exchange) before combining it with crystalline silicate. However, it is also possible to impregnate an extrudate of aluminium silicate and a binder material with a noble metal (compound) in order to prepare catalysts.

The hydrocarbon oils which are to be dewaxed with the crystalline aluminium silicate catalyst (carrier) are preferably selected from the group consisting of lubricating base oils and transformer base oils (in order to reduce their pour point), and kerosenes and gas oils (in order to reduce their freezing point).

The catalytic dewaxing process according to the present invention may suitably be carried out at a temperature of from 200-500 °C, a hydrogen pressure of from 5-100 bar, a space velocity of from 0.1-5.0 $kg.l^{-1}.h^{-1}$ and a hydrogen/oil ratio of from 100-2500 l(S.T.P.).$kg^{-1}$. The process is preferably carried out at a temperature of from 250-450 °C, a hydrogen pressure of from 10-75 bar, a space velocity of from 0.3-3 $kg.l^{-1}.h^{-1}$ and a hydrogen/oil ratio of from 200-2000 l(S.T.P.).$kg^{-1}$.

The expression "S.T.P." indicates "Standard Temperature (of 0 °C) and Pressure (of 1 bar abs.)".

Furthermore, the present invention relates to a process for separating compounds with a substantially unbranched structure from mixtures of these compounds with compounds having a branched and/or cyclic structure wherein a novel crystalline aluminium silicate as described hereinbefore, most preferably having an X-ray diffraction pattern as shown in Table A, is used which aluminium silicate has at least partly been dehydrated.

Moreover, the invention relates to a process for the preparation of liquid hydrocarbons from an olefinic hydrocarbons-containing feed wherein a catalyst is used which comprises a novel crystalline aluminium silicate as described hereinbefore, most preferably having an X-ray diffraction pattern as shown in Table A.

The invention is illustrated by the following Examples.

## EXAMPLE 1

Preparation of crystalline aluminium silicate A. An aqueous alkaline starting mixture was prepared by adding to water the following compounds: amorphous silica, aluminium sulphate, sodium sulphate, sodium hydroxide, pyridine and tetrapropyl ammonium hydroxide in such quantities that the starting mixture had the following molar composition:

93.5 $SiO_2$-1 $Al_2O_3$-30 $C_5H_5N$-0.5 $(C_3H_7)_4NOH$-7.4 $Na_2O$-19.6 $Na_2SO_4$-1938 $H_2O$.

Silicate A was prepared by maintaining the starting mixture at 150 °C for 72 hours with stirring in an autoclave under autogenous pressure. After cooling the reaction mixture crystalline aluminium silicate was filtered off, washed with water until the pH of the wash water was about 8 and dried at 120 °C for 16 hours; the dried crystalline aluminium silicate was calcined in air at 538 °C for two hours followed by an ammonium-exchange treatment and subsequently calcining in air at 500 °C for one hour to obtain the aluminium silicate in the $H^+$ form. The so obtained aluminium silicate A had the characteristic X-ray diffraction pattern given in Table A, in which "D-space" represents the interplanar spacing (in Å) calculated from the measured theta (Bragg angle) by using the Bragg equation and "$I/I_{max},\%$" represents the intensity of a peak, expressed as a percentage of the intensity of the main peak.

## TABLE A

| D-space | $I/I_{max}$,% |
|---------|---------------|
| 11.04 | 57 |
| 9.96 | 30 |
| 9.93 | 31 |
| 3.84 | 100 |
| 3.81 | 68 |
| 3.74 | 33 |
| 3.71 | 59 |
| 3.43 | 12 |
| 3.39 | 9 |
| 3.34 | 11 |
| 3.12 | 82 |
| 1.92 | 51 |

Chemical analysis of crystalline silicate A showed that its aluminium content was 1.2% by weight.

Aluminium silicate B was prepared by using a similar starting mixture as used for aluminium silicate A, except that the molar amount of $(C_3H_7)_4NOH$ was increased from 0.5 to 1.0. The starting mixture was maintained at 150 °C for 72 hours with stirring in an autoclave under autogenous pressure and treated further as described hereinabove for the preparation of aluminium silicate A.

The aluminium silicate B thus obtained gave a similar characteristic X-ray diffraction pattern as shown in Table A.

The aluminium content of aluminium silicate B was 1.2% by weight.

## COMPARATIVE EXAMPLE

Crystalline aluminium silicate C was prepared in a similar manner as aluminium silicate A by using an aqueous alkaline starting mixture having the following molar composition:

93.5 $SiO_2$ -1.25 $Al_2O_3$ -33.6 $(C_3H_7)_4NOH$ - 1.25 $Na_2O$ - 1938 $H_2O$. The characteristic X-ray diffraction pattern of crystalline aluminium silicate C is given in the following Table B.

TABLE B

| D-space | $I/I_{max}$, % |
|---------|----------------|
| 11.03 | 74 |
| 9.90 | 40 |
| 3.84 | 100 |
| 3.80 | 70 |
| 3.73 | 40 |
| 3.70 | 62 |
| 3.42 | 10 |
| 3.39 | 4 |
| 3.34 | 8 |
| 3.13 | 75 |
| 1.92 | 48 |

Aluminium silicate C, which was not in accordance with the present invention, comprised a crystalline aluminium silicate with a similar chemical composition as silicate A. However, by comparing the characteristic X-ray diffraction patterns as shown in Tables A and B it will be clear that aluminium silicate C is different from aluminium silicate A or B.

## EXAMPLE 2

Adsorption experiment with aluminium silicate A.

In Table C the (Langmuir) adsorption capacities measured at 100 °C are given.

TABLE C

| Experiment | Silicate | N-hexane, mmol/g | 2,2 dimethyl butane, mmol/g | 3 methyl pentane, mmol/g |
|------------|----------|------------------|------------------------------|--------------------------|
| 1 | A | 1.15 | 0.66 | 0.62 |

From the results given in Table C it will be clear that aluminium silicate A can be advantageously applied in a process for separating unbranched n-hexane from branched 2,2 dimethyl butane.

## Example 3

Dewaxing experiment.

A catalytic dewaxing experiment was carried out in once through operation in a reactor containing a fixed bed of the hydrogen form of aluminium silicate A diluted with silicium carbide particles.

A waxy raffinate of North Sea origin comprising 32% by weight of wax, 4.5 ppmw of sulphur and less than 1 ppmw of nitrogen, and having a pour point of +36 °C according to ASTM D97 was catalytically dewaxed in the presence of hydrogen under operating conditions given in the following Table D.

## TABLE D

### experiment 2

| | |
|---|---|
| partial hydrogen pressure, bar | 40 |
| space velocity, kg feed/l. catalyst/hour | 1.0 |
| average reactor temperature, °C | 301 |
| hydrogen feed rate, l(S.T.P.)/kg feed | 500 |

The liquid raffinate obtained in experiment 2 after catalytic dewaxing was distilled in a vigreux column; the resulting fraction boiling above 300 °C had a pour point of -18 °C, measured according to ASTM D97. The yield of liquid dewaxed raffinate, calculated as a weight percentage on basis of hydrocarbon feed, was 69.

From the reaction conditions and results shown it is clear that silicate A is an excellent dewaxing catalyst.

## EXAMPLE 4

Preparation of liquid hydrocarbons.

Propene is led with a space velocity of 0.5 kg propene/kg catalyst.hour through a microflow reactor containing 6 g of aluminium silicate A and aluminium silicate C, respectively, in the hydrogen form with a particle size of 0.2-0.6 mm at a temperature of 210 °C and a pressure of 16 bar abs.

The test results are given in the following Table E in which the selectivity after a test period of 24 hours is indicated for products having at least 5 carbon atoms per molecule ($C_5^+$). The selectivity is defined as the weight percentage of $C_5^+$ in the total product (liquid and gas).

## TABLE E

| Experiment | Catalyst | Selectivity |
|---|---|---|
| 3 | A | 99 |
| 4 (comparative) | C | 83 |

The results given in Table E clearly show that aluminium silicate A is a more selective catalyst for the preparation of liquid hydrocarbons than aluminium silicate C, which was not an aluminium silicate according to the present invention.

## Claims

1. A crystalline aluminium silicate which is obtainable by maintaining an aqueous alkaline starting mixture comprising one or more silicon compounds, one or more aluminium compounds, one or more compounds of metals from Group 1a of the Periodic Table of the Elements (MX) and organic nitrogen compounds at an elevated temperature until crystalline aluminium silicate has formed and subsequently separating crystalline silicate from the mother liquor, in which starting mixture the various compounds are present within the following molar ratios:

$RN : R_4NY = 1 - 100$,

$SiO_2 : R_4NY = 10 - 500$,

$SiO_2 : Al_2O_3 = 50 - 300$,

$SiO_2 : MX < 15$, and

$H_2O : SiO_2 = 5 - 100$,

RN representing a pyridine and $R_4NY$ representing an organic quaternary ammonium compound.

2. A crystalline aluminium silicate according to claim 1 wherein RN represents a compound selected from the group consisting of pyridine, alkyl pyridines and (substituted alkyl) pyridines.

3. A crystalline aluminium silicate according to claim 1 wherein the groups R in $R_4NY$ comprise four alkyl-groups and/or substituted alkyl-groups and Y represents an anion.

4. A crystalline aluminium silicate according to any of claims 1-3 wherein RN and $R_4NY$ are present in the starting mixture in a molar ratio from 5-80, most preferably from 10-75.

5. A crystalline aluminium silicate having an X-ray diffraction pattern substantially as shown in Table A.

6. A crystalline aluminium silicate substantially as described hereinbefore with reference to Examples 1 and 2.

7. Process for the preparation of a crystalline aluminium silicate according to any of claims 1-6 which comprises maintaining an aqueous alkaline starting mixture comprising one or more silicon compounds, one or more aluminium compounds, one or more compounds of metals from Group 1a of the Periodic Table of the Elements (MX) and organic nitrogen compounds at an elevated temperature until crystalline aluminium silicate has formed and subsequently separating crystalline silicate from the mother liquor, in which starting mixture the various compounds are present within the following molar ratios:

$RN : R_4NY = 1 - 100$,

$SiO_2 : R_4NY = 10 - 500$,

$SiO_2 : Al_2O_3 = 50 - 300$,

$SiO_2 : MX < 15$, and

$H_2O : SiO_2 = 5 - 100$

RN representing a pyridine and $R_4NY$ representing an organic quaternary ammonium compound.

8. Process for the catalytic dewaxing of hydrocarbon oils wherein the catalyst used comprises a crystalline aluminium silicate according to any of claims 1-6.

9. Process for separating compounds with a substantially unbranched structure from mixtures of these compounds with compounds having a branched and/or cyclic structure wherein a crystalline aluminium silicate according to any of claims 1-6 is used which aluminium silicate has at least partly been dehydrated.

10. Process for the preparation of liquid hydrocarbons from an olefinic hydrocarbons-containing feed wherein a catalyst is used which comprises a crystalline aluminium silicate according to any of claims 1-6.

8